# EUROPEAN PATENT APPLICATION

(11) **EP 4 325 414 A1**
(43) Date of publication of application: **21.02.2024**
(21) Application number: 23191325.2
(22) Date of filing: 14.08.2023
(51) Int. Cl.: G06Q 30/0601, G06Q 50/22, G06Q 20/14, G06Q 10/02

(54) **CONTROL METHOD, APPARATUS, SERVER, AND STORAGE MEDIUM FOR A CRYOGENIC PHYSICAL THERAPY SYSTEM**

(30) Priority: 15.08.2022 CN 202210974230
(71) Applicant: Long, Zhigang, 100085 Beijing (CN)
(72) Inventor: Long, Zhigang, 100085 Beijing (CN)
(74) Representative: Piticco, Lorena

(57) **Abstract**

The embodiments of the present invention provide a control method, apparatus, server, and storage medium for a cryogenic physical therapy system. The method is applied to a server in the cryogenic physical therapy system, including: acquiring target location information input by a user, and recommending a cryogenic physical therapy device to the user based on a relationship between the target location information and pre-recorded location information of each of cryogenic physical therapy devices; determining a target cryogenic physical therapy device selected by the user and determining whether an account balance of the user meets a preset fee deduction condition; if the account balance meets the preset fee deduction condition, sending a use request to the target cryogenic physical therapy device to enable the target cryogenic physical therapy device to control a target component to perform an operation corresponding to the use request; receiving a fee deduction request sent by the target cryogenic physical therapy device and updating the account balance of the user based on the fee deduction request. Therefore, the user can select a suitable cryogenic physical therapy device according to actual situations to perform cryogenic physical therapy quickly.

## Description

### Technical Field

The present application relates to the technical field of cryogenic physical therapy, in particular to a control method, apparatus, server, and storage medium for a cryogenic physical therapy system.

### Background

With the acceleration of modern life rhythm, more and more people are in sub-health state, and it is extremely unfavorable to human health to be in sub-health state for a long time. As an emerging physiotherapy technology, cryogenic physical therapy technology is gradually getting into the field of vision of the masses from the traditional medical field. The cryogenic physical therapy is the process of exposing the human body to an extremely low temperature (usually below -110 °C) environment for a period of time, such as about 2-3 minutes, and stimulate the skin of a user through low temperature, so that endorphin is released in the body of the user, and the functional effects of accelerating blood circulation, continuously burning fat, increasing skin elasticity, and eliminating muscle fatigue and the like are achieved.

Due to the high selling price and maintenance cost of a cryogenic physical therapy device, a service merchant of the cryogenic physical therapy device usually installs the cryogenic physical therapy device in a certain fixed area to reduce operational service costs. When a user wants to perform cryogenic physical therapy, the user first needs to go to the area and recharge or apply for member services at the service merchant before starting to perform cryogenic physical therapy.

The user can use the amount of money recharged or the applied member service at a service merchant of a certain cryogenic physical therapy device only at this service merchant. Therefore, if the user wants to perform the cryogenic physical therapy again in the future, he/she needs to go to the service merchant. If the user is far away from the service merchant, it is very inconvenient to perform the cryogenic physical therapy. It is difficult for the user to select a suitable cryogenic physical therapy device according to actual situations to perform cryogenic physical therapy quickly.

### Summary

The purpose of the embodiments of the present invention is to provide a control method, apparatus, server, and storage medium for a cryogenic physical therapy system, so that the user can select a suitable cryogenic physical therapy device according to actual situations to perform cryogenic physical therapy quickly. Specifically, the technical solutions are as follows:
In a first aspect, an embodiment of the present invention provides a control method for a cryogenic physical therapy system, wherein the method is applied to a server in the cryogenic physical therapy system, the cryogenic physical therapy system further comprises cryogenic physical therapy devices, the method includes:
acquiring target location information input by a user, and recommending a cryogenic physical therapy device to the user based on a relationship between the target location information and pre-recorded location information of each of the cryogenic physical therapy devices;
determining a target cryogenic physical therapy device selected by the user and determining whether an account balance of the user meets a preset fee deduction condition;
if the account balance meets the preset fee deduction condition, sending a use request to the target cryogenic physical therapy device to enable the target cryogenic physical therapy device to control a target component to perform an operation corresponding to the use request, wherein the use request comprises a start time, a duration, and a physical therapy temperature;
receiving a fee deduction request sent by the target cryogenic physical therapy device and updating the account balance of the user based on the fee deduction request.

Optionally, the step of recommending a cryogenic physical therapy device to the user based on a relationship between the target location information and pre-recorded location information of each of the cryogenic physical therapy devices include:
acquiring an operating state of each of the cryogenic physical therapy devices, wherein the operating state comprises a working state, a reserved state, and a standby state;
recommending a cryogenic physical therapy device whose operating state is the standby state to the user based on a relationship between the target location information and pre-recorded location information of each of the cryogenic physical therapy devices.

Optionally, the method further includes:
acquiring operational information provided by a merchant of each of the cryogenic physical therapy devices, wherein the operational information comprises business hours and prices for physical therapy of this cryogenic physical therapy device;
the method further comprises:
   when recommending a cryogenic physical therapy device to the user, displaying the operational information corresponding to the recommended cryogenic physical therapy device.

Optionally, the server pre-records authentication information and an account balance of each user, and the method further includes:
acquiring feature information and/or recharge information provided by the user;
updating the authentication information of the user based on the feature information, and updating the account balance of the user based on the recharge information;
after the step of determining a target cryogenic physical therapy device selected by the user, the method further comprises:
   if the account balance meets the preset fee deduction condition, sending the authentication information of the user to the target cryogenic physical therapy device to enable the target cryogenic physical therapy device to authenticate a target user.

Optionally, the method further includes:
receiving a prompt message sent by a cryogenic physical therapy device, wherein the prompt message is generated when the cryogenic physical therapy device determines that an abnormal situation occurs based on a device state;
generating a repair request based on the prompt message and sending the repair request to a device manufacturer of the cryogenic physical therapy device.

Optionally, the method further includes:
when the account balance of the user is updated based on the fee deduction request, performing income distribution between a merchant of the target cryogenic physical therapy device and a device manufacturer of the target cryogenic physical therapy device according to a preset ratio.

In a second aspect, an embodiment of the present invention provides a control method for a cryogenic physical therapy system, wherein the method is applied to a cryogenic physical therapy device in the cryogenic physical therapy system, the cryogenic physical therapy system further comprises a server, the method includes:
receiving a use request sent by the server, wherein the use request comprises a start time, a duration, and a physical therapy temperature;
controlling a target component to perform an operation corresponding to the use request;
when determining that the user has completed cryogenic physical therapy, sending a fee deduction request to the server.

Optionally, the step of controlling a target component to perform an operation corresponding to the use request includes:
receiving authentication information of the user sent by the server;
acquiring target feature information of a target user and determining whether the target feature information matches the authentication information;
if the target feature information matches the authentication information, controlling the target component to perform the operation corresponding to the use request.

Optionally, the method further includes:
acquiring a device state and determining whether an abnormal situation occurs based on the device state;
if an abnormal situation occurs, generating a prompt message based on the abnormal situation and sending the prompt message to the server.

In a third aspect, an embodiment of the present invention provides a control apparatus for a cryogenic physical therapy apparatus, wherein the apparatus is applied to a server in the cryogenic physical therapy system, the cryogenic physical therapy system further comprises cryogenic physical therapy devices, the apparatus includes:
a recommendation module, configured for acquiring target location information input by a user, and recommending a cryogenic physical therapy device to the user based on a relationship between the target location information and pre-recorded location information of each of the cryogenic physical therapy devices;
a balance determining module, configured for determining a target cryogenic physical therapy device selected by the user and determining whether an account balance of the user meets a preset fee deduction condition;
a use request sending module, configured for sending, if the account balance meets the preset fee deduction condition, a use request to the target cryogenic physical therapy device to enable the target cryogenic physical therapy device to control a target component to perform an operation corresponding to the use request, wherein the use request comprises a start time, a duration, and a physical therapy temperature;
a balance updating module, configured for receiving a fee deduction request sent by the target cryogenic physical therapy device and updating the account balance of the user based on the fee deduction request.

Optionally, the recommendation module includes:
an operating state acquisition unit, configured for acquiring an operating state of each of the cryogenic physical therapy devices, wherein the operating state comprises a working state, a reserved state, and a standby state;
a first recommendation unit, configured for recommending a cryogenic physical therapy device whose operating state is the standby state to the user based on a relationship between the target location information and pre-recorded location information of each of the cryogenic physical therapy devices.

Optionally, the apparatus further includes:
an operational information acquisition module, configured for acquiring operational information provided by a merchant of each of the cryogenic physical therapy devices, wherein the operational information comprises business hours and prices for physical therapy of this cryogenic physical therapy device;
the apparatus further includes:
   a second recommendation unit, configured for, when recommending a cryogenic physical therapy device to the user, displaying the operational information corresponding to the recommended cryogenic physical therapy device.

Optionally, the server pre-records authentication information and an account balance of each user, and the apparatus further includes:
an user information acquisition module, configured for acquiring feature information and/or recharge information provided by the user;
an user information update module, configured for updating the authentication information of the user based on the feature information, and updating the account balance of the user based on the recharge information;
the apparatus further includes:
   an authentication information sending unit, configured for sending, if the account balance meets the preset fee deduction condition, authentication information of the user to the target cryogenic physical therapy device to enable the target cryogenic physical therapy device to authenticate a target user after determining the target cryogenic physical therapy device selected by the user.

Optionally, the apparatus further includes:
a prompt message acquisition module, configured for receiving a prompt message sent by a cryogenic physical therapy device, wherein the prompt message is generated when the cryogenic physical therapy device determines that an abnormal situation occurs based on a device state;
a repair request sending module, configured for generating a repair request based on the prompt message and sending the repair request to a device manufacturer of the cryogenic physical therapy device.

Optionally, the apparatus further includes:
an income distribution module, configured for, when the account balance of the user is updated based on the fee deduction request, performing income distribution between a merchant of the target cryogenic physical therapy device and a device manufacturer of the target cryogenic physical therapy device according to a preset ratio.

In a fourth aspect, an embodiment of the present invention provides a control apparatus for a cryogenic physical therapy system, wherein the apparatus is applied to a cryogenic physical therapy device in the cryogenic physical therapy system, the cryogenic physical therapy system further comprises a server, the apparatus includes:
a use request receiving module, configured for receiving a use request sent by the server, wherein the use request includes a start time, a duration, and a physical therapy temperature;
an operation performing module, configured for controlling a target component to perform an operation corresponding to the use request;
a fee deduction request sending module, configured for, when determining that a user has completed cryogenic physical therapy, sending a fee deduction request to the server.

Optionally, the operation performing module includes:
an authentication information receiving unit, configured for receiving authentication information of the user sent by the server;
an information determining unit, configured for acquiring target feature information of a target user and determining whether the target feature information matches the authentication information;
an operation performing unit, configured for controlling, if the target feature information matches the authentication information, the target component to perform the operation corresponding to the use request.

Optionally, the apparatus further comprises:
an abnormal situation determining module, configured for acquiring a device state and determining whether an abnormal situation occurs based on the device state;
a prompt message sending module, configured for generating, if an abnormal situation has occurred, a prompt message based on the abnormal situation and sending the prompt message to the server.

In a fifth aspect, an embodiment of the present invention provides a server comprising a processor, a communications interface, a memory and a communication bus, wherein the processor, the communications interface, and the memory communicate with each other via the communication bus;
the memory is provided for storing a computer program;
the processor is used for implementing the method of any one of the first aspect described above when executing the program stored on the memory.

In a sixth aspect, an embodiment of the disclosure provides a computer readable storage medium, wherein the computer readable storage medium stores a computer program thereon, the method of any one of the first aspect or the second aspect described above is implemented when the computer program is executed by a processor.

The beneficial effects of the embodiments of the present invention are as follows:
In the solution provided by the embodiments of the present invention, the server can acquire target location information input by a user, and recommend a cryogenic physical therapy device to the user based on a relationship between the target location information and pre-recorded location information of each of the cryogenic physical therapy devices; determine a target cryogenic physical therapy device selected by the user and determine whether an account balance of the user meets a preset fee deduction condition. If the account balance meets the preset fee deduction condition, it can send a use request to the target cryogenic physical therapy device to enable the target cryogenic physical therapy device to control a target component to perform an operation corresponding to the use request, wherein the use request comprises a start time, a duration, and a physical therapy temperature. Furthermore, it can receive a fee deduction request sent by the target cryogenic physical therapy device and update the account balance of the user based on the fee deduction request. It can be seen that due to the pre-recorded location information of each of the cryogenic physical therapy devices, the server can recommend a cryogenic physical therapy device that is closer to the target location information after acquiring target location information input by a user, and the server can update the account balance of the user after completing the cryogenic physical therapy. Therefore, the user does not need to go to a specific service merchant and can select a suitable cryogenic physical therapy device according to actual situations to perform cryogenic physical therapy quickly. Of course, implementing any product or method of the present invention does not necessarily require simultaneously achieving all the advantages described above.

### Brief Description of the Drawings

In order to explain the technical solutions of embodiments of the present invention or of the prior art, a simple introduction of the drawings required in the description of the embodiments and of prior art will be given. Obviously, the drawings described below are just those of some embodiments of the present invention and other embodiments may be obtained by those of ordinary skill in the art.
Fig. 1 is a flowchart of a control method for a cryogenic physical therapy system provided by an embodiment of the present invention;
Fig. 2 is a specific flowchart of step S101 according to the embodiment shown in Fig. 1;
Fig. 3 is a flowchart for updating authentication information and account balance according to the embodiment shown in Fig. 1;
Fig. 4 is a flowchart for sending a repair request according to the embodiment shown in Fig. 1;
Fig. 5 is a flowchart of another control method for the cryogenic physical therapy system provided by an embodiment of the present invention;
Fig. 6 is a specific flowchart of step S502 according to the embodiment shown in Fig. 5;
Fig. 7 is a flowchart for sending a prompt message according to the embodiment shown in Fig. 5;
Figure 8 is a structural schematic diagram of a control apparatus for the cryogenic physical therapy system provided by an embodiment of the present invention;
Fig. 9 is a structural schematic diagram of another control apparatus of the cryogenic physical therapy system provided by an embodiment of the present invention;
Fig. 10 is a structural schematic diagram of a server provided by an embodiment of the present invention.

### Detailed Description

The technical solutions of the disclosure will be described in detail with reference to the drawings of embodiments of the disclosure. Obviously, the embodiments described are merely parts of the embodiments of the disclosure, instead of all the embodiments. All other embodiments obtained by those of ordinary skill in the art based on the present application are within the scope of the disclosure.

In order to achieve that the user can select a suitable cryogenic physical therapy device according to actual situations to perform cryogenic physical therapy quickly, the embodiments of the present application provide a control method, apparatus, server, and storage medium for a cryogenic physical therapy system, a control method for a cryogenic physical therapy system provided by an embodiment of the present invention is introduced firstly as follows.

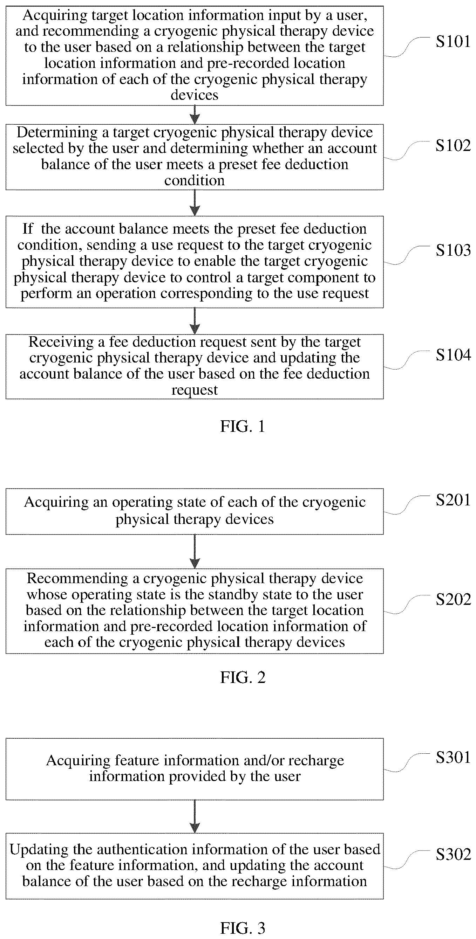

As shown in Fig. 1, a control method for a cryogenic physical therapy system is applied to a server in the cryogenic physical therapy system, the cryogenic physical therapy system further comprises cryogenic physical therapy devices, the method includes:
S 101, acquiring target location information input by a user, and recommending a cryogenic physical therapy device to the user based on a relationship between the target location information and pre-recorded location information of each of the cryogenic physical therapy devices.
S 102, determining a target cryogenic physical therapy device selected by the user and determining whether an account balance of the user meets a preset fee deduction condition.
5 103, if the account balance meets the preset fee deduction condition, sending a use request to the target cryogenic physical therapy device to enable the target cryogenic physical therapy device to control a target component to perform an operation corresponding to the use request;
   wherein the use request comprises a start time, a duration, and a physical therapy temperature.
5 104, receiving a fee deduction request sent by the target cryogenic physical therapy device and updating the account balance of the user based on the fee deduction request.

It can be seen that in the solution provided by the embodiment of the present invention, the server can acquire target location information input by a user, and recommend a cryogenic physical therapy device to the user based on a relationship between the target location information and pre-recorded location information of each of the cryogenic physical therapy devices; determine a target cryogenic physical therapy device selected by the user and determine whether an account balance of the user meets a preset fee deduction condition. If the account balance meets the preset fee deduction condition, a use request can be sent to the target cryogenic physical therapy device, wherein the use request includes a start time, a duration, and a physical therapy temperature. Furthermore, it can receive a fee deduction request sent by the target cryogenic physical therapy device and update the account balance of the user based on the fee deduction request. It can be seen that due to the pre-recorded location information of each of the cryogenic physical therapy devices, the server can recommend a cryogenic physical therapy device that is closer to target location information after acquiring the target location information input by a user, and the server can update the account balance of the user after completing the cryogenic physical therapy. Therefore, the user does not need to go to a specific service merchant and can select a suitable cryogenic physical therapy device according to actual situations to perform cryogenic physical therapy quickly.

In order to enable user to select a suitable cryogenic physical therapy device based on their actual situation without going to a specific service merchant, in step S101 above, the server can acquire target location information input by a user and recommend a cryogenic physical therapy device to the user based on a relationship between the target location information and pre-recorded location information of each of the cryogenic physical therapy devices.

When the user wants to perform the cryogenic physical therapy, he/she can input the target location information to the server, the target location information can be represented as the target location for the user to perform the cryogenic physical therapy. For example, when the user is at home, the input target location information can be location information of a location where the user is located, so that the user can perform the cryogenic physical therapy on a cryogenic physical therapy device near the home. In one implementation, the user can also input the target location information and an appointment time for cryogenic physical therapy, so that the user can go to a location corresponding to the target location information within the appointment time to perform the cryogenic physical therapy. For example, when a user wants to perform the cryogenic physical therapy during a business trip, he/she can input a target location information during business trip and a time for reaching the target location to complete a cryogenic physical therapy appointment. Then, after the current time reaches the appointment time, the user can go to the cryogenic physical therapy device at the target location for performing cryogenic physical therapy.

The manner in which the user inputs target location information to the server can include: going to a merchant of the cryogenic physical therapy device to input the target location information, or inputting the target location information through a smart device associated with the server. The smart device can establish a communication connection with the server through 4G network connection, 5G network connection, and WIFI (Wireless Fidelity) connection. For example, the smart device can be an electronic device such as a mobile phone, a tablet computer, and a computer, and then through an application client on the smart device, the user can more conveniently select a target location for cryogenic physical therapy, which is not specifically limited herein.

The server can pre-record location information of each of the cryogenic physical therapy devices, the location information can be location information provided when each of the cryogenic physical therapy devices is installed for the first time. When the cryogenic physical therapy device changes its installation location, the location information recorded by the server can also be updated accordingly. In one implementation, each cryogenic physical therapy device may also include a positioning apparatus, which can acquire positioning information of the cryogenic physical therapy device in real time and send the positioning information to the server, so that the server can update location information of the respective cryogenic physical therapy devices in real time.

After acquiring the target location information input by the user, because the location information of each of the cryogenic physical therapy devices is recorded in advance, the server can recommend a cryogenic physical therapy device to the user based on the relationship between the target location information and the pre-recorded location information of each of the cryogenic physical therapy devices. The server can recommend a cryogenic physical therapy device that is closer to the target location information to the user; it can also recommend a cryogenic physical therapy device that the user may be interested in based on the history of cryogenic physical therapy of the user, for example, it can provide cryogenic physical therapy devices with different additional services for different prices.

Furthermore, in the above step S102, the server can determine a target cryogenic physical therapy device selected by the user and determine whether an account balance of the user meets a preset fee deduction condition.

When the user selects a cryogenic physical therapy device, the server can use the cryogenic physical therapy device as the target cryogenic physical therapy device. Because the account balance of each user can be recorded in advance, the server can determine whether the account balance of the user meets a preset fee deduction condition. Wherein, the preset fee deduction condition can be comprehensively considered based on conditions such as the different cryogenic physical therapy device selected by the user, the duration of cryogenic physical therapy, and whether the appointment time is during a use peak period, etc., which is not limited here. In one implementation, the merchant of cryogenic physical therapy device can also provide member services, and the server can record member service information of each user. For example, the member service information can include information such as the remaining usage times within the member validity period of the member, etc., making it more convenient for the user to perform cryogenic physical therapy.

If the account balance meets the preset fee deduction condition, the server can send a use request to the target cryogenic physical therapy device to enable the target cryogenic physical therapy device to control a target component to perform an operation corresponding to the use request, that is, execute step S103.

If the account balance of the user meets the preset fee deduction condition, the server can confirm that this transaction can proceed, and then it can send a use request to the target cryogenic physical therapy device to enable the target cryogenic physical therapy device to control a target component to perform an operation corresponding to the use request when receiving the use request. The use request can include a start time, a duration, and a physical therapy temperature. The target cryogenic physical therapy device can control the target component to lower the temperature to reach the physical therapy temperature when the current time reaches the start time, and continuously maintain the temperature to be the physical therapy temperature within the duration, so that user can perform cryogenic physical therapy in the target cryogenic physical therapy device.

Until the user's cryogenic physical therapy is finished, the target cryogenic physical therapy device can confirm that the physiotherapy of the user is completed and send a fee deduction request to the server. Furthermore, in the step S104 above, the server can receive the fee deduction request sent by the target cryogenic physical therapy device and update the account balance of the user based on the fee deduction request.

The server can update the account balance of the user based on the fee deduction request, that is, perform a fee deduction operation on the account balance of the user. The amount of the fee deduction operation is the amount indicated by the fee deduction condition when the user selects the target cryogenic physical therapy device. In one implementation, the server can record the member service information of each user. When the member service information includes information such as the remaining usage times within the member validity period of the member, etc., the server can also update the remaining usage times of the user based on the fee deduction request.

It can be seen that using the solution provided by the embodiment of the present invention, due to the server pre-records location information of each of the cryogenic physical therapy devices, the server can recommend a cryogenic physical therapy device that is closer to the target location information after acquiring target location information input by a user, and the server can update the account balance of the user after completing the cryogenic physical therapy. Therefore, the user does not need to go to a specific service merchant and can select a suitable cryogenic physical therapy device according to actual situations to perform cryogenic physical therapy quickly. A cryogenic physical therapy device can be provided to different users at different time periods, thereby reducing the requirements for cryogenic physical therapy and better meeting the cryogenic physical therapy needs of more and more users.

As an implementation of the present invention, as shown in Fig. 2, the step of recommending a cryogenic physical therapy device to the user based on a relationship between the target location information and pre-recorded location information of each of the cryogenic physical therapy devices can include:
S201, acquiring an operating state of each of the cryogenic physical therapy devices;
wherein, the operating state includes a working state, a reserved state, and a standby state.

In order to reduce waiting time of a user and quickly perform cryogenic physical therapy, the server can acquire the operating state of each cryogenic physical therapy device. In one implementation, a cryogenic physical therapy device may include a sensing apparatus, which may include various types of sensing devices, such as a temperature sensor, a humidity sensor, a human body sensor, a timer, a camera, etc., which are not particularly limited herein. The sensing apparatus may acquire internal environmental data of the cryogenic physical therapy device, including temperature data acquired by the temperature sensor, humidity data acquired by the humidity sensor, human body data detected by the human body sensor, monitoring videos acquired by the camera, and cryogenic physical therapy duration acquired by the timer.

The sensing apparatus can send the acquired internal environmental data of the cryogenic physical therapy device to the server. Based on this internal environmental data, the server can determine the operating state of each cryogenic physical therapy device, which can include a working state, a reserved state, and a standby state. For example, when the data acquired by the sensing apparatus includes temperature data acquired by the temperature sensor which is -110 °C, and when the human body sensor detects the presence of a user inside the cryogenic physical therapy device, the server can determine the operating state of the cryogenic physical therapy device as the working state based on the above data. For example, when the data acquired by the sensing apparatus includes temperature data acquired by the temperature sensor which is -30°C, and the human body sensor detects that there is no user inside the cryogenic physical therapy device, and the server determines that current time does not reach the start time of the cryogenic physical therapy corresponding to the use request, the server can determine the operating state of the cryogenic physical therapy device as the reserved state.

S202, recommending a cryogenic physical therapy device whose operating state is the standby state to the user based on the relationship between the target location information and pre-recorded location information of each of the cryogenic physical therapy devices.

Furthermore, the server can record and update the operating state of each cryogenic physical therapy device, and when it recommends a cryogenic physical therapy device to a user, it recommends a cryogenic physical therapy device that is closer to the target location information and whose operating state is the standby state, thereby reducing waiting time of the user and allowing the user to quickly perform cryogenic physical therapy.

It can be seen that in this embodiment, the server can acquire the operating state of each cryogenic physical therapy device and recommend a cryogenic physical therapy device whose operating state is the standby state to the user based on the relationship between the target location information and the pre-recorded location information of each of the cryogenic physical therapy devices. This reduces waiting time of the user and greatly improves user experience, allowing the user to perform cryogenic physical therapy more quickly.

As one implementation of the present invention, the above method may further include:
acquiring operational information provided by a merchant of each of the cryogenic physical therapy devices;
wherein, the operational information includes business hours and prices for physical therapy of this cryogenic physical therapy device.

In order to better recommend a suitable cryogenic physical therapy device to a user and meet the cryogenic physical therapy needs of different users, the server can acquire operational information provided by the merchant of each of the cryogenic physical therapy devices. In one implementation, the merchant of each cryogenic physical therapy device can provide operational information to the server through a smart device associated with the server. The smart device can establish a communication connection with the server through 4G network connection, 5G network connection, and WIFI connection, which can be a computer or server installed with a remote client of the cryogenic physical therapy system; a mobile phone or tablet installed with a software application corresponding to the cryogenic physical therapy system, etc., which is not limited here.

The operational information can include business hours of the cryogenic physical therapy device to meet physiotherapy needs of cryogenic physical therapy users with different time habits. The operational information can also include prices for physical therapy of the cryogenic physical therapy device. The merchant of the cryogenic physical therapy device can also reasonably set the prices for physical therapy based on conditions such as different types of cryogenic physical therapy devices, duration for cryogenic physical therapy, etc., thereby meeting the cryogenic physical therapy needs of users with different consumer needs.

The above method can also include:
when recommending a cryogenic physical therapy device to the user, displaying the operational information corresponding to the recommended cryogenic physical therapy device.

Therefore, when the server recommends a cryogenic physical therapy device to the user, it can display the corresponding operational information of the recommended cryogenic physical therapy device, so that user can not only know distances from the cryogenic physical therapy devices to the selected target location, but also determine which cryogenic physical therapy device is in business state near the target location, as well as prices for physical therapy of this cryogenic physical therapy device, so that the user can more conveniently select the target cryogenic physical therapy device that meets its own needs.

It can be seen that in this embodiment, the server can acquire operational information provided by the merchant of each cryogenic physical therapy device, and display the operational information corresponding to the recommended cryogenic physical therapy device when recommending the cryogenic physical therapy device to the user. Thus, the user can select a suitable target cryogenic physical therapy device more conveniently based on its actual situation.

As an implementation of the present invention, as shown in Fig. 3, the above server can pre-record the authentication information and account balance of each user, and the above method can also include:
S301, acquiring feature information and/or recharge information provided by the user.

In order to avoid use disputes caused by persons other than the user entering the cryogenic physical therapy device, or the risk of low-temperature frostbite caused by other persons mistakenly entering the cryogenic physical therapy device, the server can record the authentication information of each user. The authentication information is used to authenticate the person entering the target cryogenic physical therapy device, which can specifically be biometric information of the user, including facial feature information, fingerprint feature information, iris feature information, etc., which is not specifically limited herein. In one implementation, when a user first connects to a server through a merchant of a cryogenic physical therapy device or a smart device installed with a client of the cryogenic physical therapy device, an account of the user can be registered, and the facial feature information, fingerprint feature information, iris feature information, and other information can be acquired through the merchant or smart device of the cryogenic physical therapy device. The server can acquire and record the biometric information as authentication information of the user.

In one implementation, the authentication information may also include password information provided by the server to the user in advance, information corresponding to an IC (Integrated Circuit) card, information corresponding to an RDFI (Radio Frequency Identification) tag, etc., which is not limited herein.

In order to facilitate the user to perform cryogenic physical therapy device according to its own needs, the user can carry out cash recharging through a merchant of the cryogenic physical therapy device, or carry out online recharging or recharging member service on the account of the user through a smart device associated with the server, and then the smart device can generate recharge information. The recharge information can include changes of the user's account balance, or changes of the user's member duration, available usage times, and other information.

S302, updating the authentication information of the user based on the feature information, and updating the account balance of the user based on the recharge information.

Furthermore, the server can update the authentication information of the user based on the acquired feature information, and update the account balance of the user based on the recharge information.

After the step of determining a target cryogenic physical therapy device selected by the user, the above method may further include:
if the account balance meets the preset fee deduction condition, sending the authentication information of the user to the target cryogenic physical therapy device to enable the target cryogenic physical therapy device to authenticate a target user.

If the account balance of the user meets the preset fee deduction condition, the server can send the authentication information of the user to the target cryogenic physical therapy device. The authentication information can include the user's facial feature information, fingerprint feature information, iris feature information, etc., it can also include password information provided by the server to the user in advance, information corresponding to the IC card, information corresponding to the RDFI tag, etc.

The target user may be a person who wants to use the target cryogenic physical therapy device, including the user. The target cryogenic physical therapy device can authenticate the target user based on this authentication information. For example, when facial feature information of the target user is the same as the authentication information, or when a password input by the target user is the same as the password provided by the server in advance, or when an IC card or a RDFI tag carried by the target user matches the authentication information, the target user can be considered as the user, and the target cryogenic physical therapy device can confirm that the authentication has been passed, and then the target component can be controlled to perform the operation corresponding to the use request. When the authentication fails, the target cryogenic physical therapy device does not control the target component to perform the operation corresponding to the use request.

It can be seen that in this embodiment, the server can acquire feature information provided by the user and acquire recharge information provided by the user; and update the authentication information of the user based on the feature information, and update the account balance of the user based on the recharge information. Thus, the user does not need to go to the merchant of cryogenic physical therapy device for operations such as updating authentication information and recharging. The user can easily and quickly complete operations such as updating authentication information and recharging through a first user device connected to the server network. If the account balance meets the preset fee deduction condition, the server can also send the authentication information of the user to the target cryogenic physical therapy device to enable the target cryogenic physical therapy device to authenticate a target user. This can avoid the use disputes caused by that people other than the user entering cryogenic physical therapy device, or the risk of low-temperature frostbite.

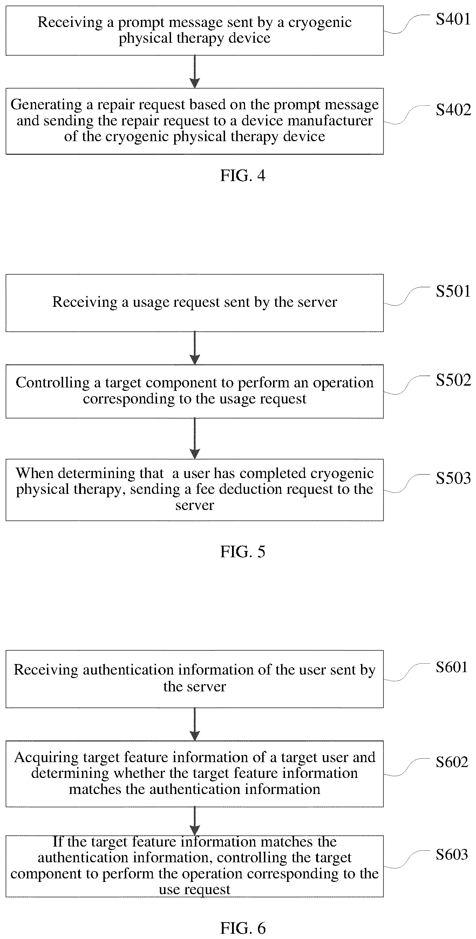

As one implementation of the present invention, as shown in Fig. 4, the above method may further include:
S401, receiving a prompt message sent by the cryogenic physical therapy device.

Wherein, the prompt message is generated when the cryogenic physical therapy device determines that an abnormal situation occurs based on a device state.

The cryogenic physical therapy device can acquire its own device state, which can include device information of the cryogenic physical therapy device, such as location information, device usage times, device fault information, etc. In one implementation, the device state can also include sensing data of the internal environment of the cryogenic physical therapy device acquired by the sensing apparatus, such as temperature data, humidity data, human body data, monitoring videos, cryogenic physical therapy duration, and other data, which is not specifically limited here. Furthermore, the cryogenic physical therapy device can determine whether an abnormal situation occurs, and the abnormal situation can include prolonged physiotherapy time for the user within the cryogenic physical therapy device, circuit failure of the cryogenic physical therapy device, etc., which is not specifically limited here.

When the cryogenic physical therapy device determines that an abnormal situation occurs, it can generate a prompt message based on the abnormal situation and send the prompt message to the server. Thus, the server can receive the prompt message sent by the cryogenic physical therapy device.

S402, generating a repair request based on the prompt message and sending the repair request to a device manufacturer of the cryogenic physical therapy device.

Based on the received prompt message, the server can generate a corresponding repair request and send the repair request to a device manufacturer of the cryogenic physical therapy device. For example, when the abnormal situation is that a user has been in a cryogenic physical therapy device for too long, the repair request can be "the user has been in a cryogenic physical therapy device A for too long, please turn off the cryogenic physical therapy device A", so that the device manufacturer of the cryogenic physical therapy device can remotely force the shutdown of the cryogenic physical therapy device based on the repair request. For example, when the abnormal situation is a circuit failure of a cryogenic physical therapy device, the repair request can include location information of the cryogenic physical therapy device with the circuit failure, as well as a specific location of the circuit failure, so that the device manufacturer can quickly arrange the staff to perform device repair.

It can be seen that in this embodiment, the server can receive a prompt message sent by the cryogenic physical therapy device, generate a repair request based on the prompt message, and send the repair request to the device manufacturer of the cryogenic physical therapy device. Therefore, the device manufacturer of the cryogenic physical therapy device can quickly arrange the staff to carry out personnel rescue and device repair based on the repair request, in order to protect the physical health of the user and the property safety of merchants.

As one implementation of the present invention, the above method may further include:
when the account balance of the user is updated based on the fee deduction request, performing income distribution between the merchant of the cryogenic physical therapy device and the device manufacturer of the cryogenic physical therapy device according to a preset ratio.

Due to the high price of the cryogenic physical therapy device and the high cost of one-time investment by the merchant, there are difficulties in promoting the cryogenic physical therapy device. The merchant of the cryogenic physical therapy device can operate the cryogenic physical therapy device with lower investment costs through leasing and selling, thereby reducing the investment funds of the merchant of the cryogenic physical therapy device.

The merchant of the cryogenic physical therapy device can pre-set an income distribution ratio with the device manufacturer. In this way, after the cryogenic physical therapy of the user is completed, the server can update the account balance of the user based on the fee deduction request. The cryogenic physical therapy consumption of the user can be distributed between the merchant of the cryogenic physical therapy device and the device manufacturer of the cryogenic physical therapy device according to a preset ratio.

It can be seen that in this embodiment, the server can update the account balance of the user based on the fee deduction request and perform income distribution between the merchant of the cryogenic physical therapy device and the device manufacturer of the cryogenic physical therapy device according to the preset ratio. This reduces the investment of the merchant of the cryogenic physical therapy device, which is conducive to the promotion of cryogenic physical therapy and can better meet the needs of users for cryogenic physical therapy.

The following is an introduction to another control method of a cryogenic physical therapy system provided by an embodiment of the present invention, as shown in Fig. 5, a control method of a cryogenic physical therapy system is applied to a cryogenic physical therapy device in the cryogenic physical therapy system, and the cryogenic physical therapy system further includes a server. The method includes:
S501, receiving a use request sent by the server;
wherein the use request comprises a start time, a duration, and a physical therapy temperature.

S502, controlling a target component to perform an operation corresponding to the use request.

S503, when determining that the user has completed cryogenic physical therapy, sending a fee deduction request to the server.

It can be seen that in the solution provided by the embodiment of the present invention, the cryogenic physical therapy device can receive a use request sent by the server, control the target component to perform an operation corresponding to the use request, and send a fee deduction request to the server when determining that the cryogenic physical therapy has been completed by the user. After selecting a suitable cryogenic physical therapy device according to the actual situation, the cryogenic physical therapy device can control the target component to create an extremely low-temperature environment required for cryogenic physical therapy, and send a fee deduction request to the server when the cryogenic physical therapy has been completed by the user, thereby updating the account balance of the user. Therefore, the user does not need to go to a specific service merchant and can select a suitable cryogenic physical therapy device according to actual situations to perform cryogenic physical therapy quickly.

In the above step S501, the cryogenic physical therapy device can receive a use request sent by the server, wherein the use request includes a start time, a duration, and a physical therapy temperature. Based on the received use request, the cryogenic physical therapy device can generate the extremely low-temperature environment required for cryogenic physical therapy corresponding to the physical therapy temperature after the current time reaches the start time, and maintain the preset duration.

The cryogenic physical therapy device can control the target component to perform an operation corresponding to the use request, that is, execute step S502 above. That is, the cryogenic physical therapy device can control the target component to perform the operation corresponding to the use request, generate the extremely low-temperature environment required for cryogenic physical therapy corresponding to the physical therapy temperature after the current time reaches the start time, and maintain the preset duration.

The target component can include an electric cabin door, a refrigeration apparatus and a heating apparatus, etc. The electric cabin door is used to control a user to get in and out of the cryogenic physical therapy device and maintain the internal temperature of the cryogenic physical therapy device. The refrigeration apparatus is an apparatus used to reduce the internal temperature of the cryogenic physical therapy device, which can provide the user with the extremely low-temperature environment required for cryogenic physical therapy. The refrigeration apparatus can have various refrigeration modes, for example, electricity can be used to use a compressor to charge refrigerant for refrigeration; liquid nitrogen vaporization can also be used for refrigeration, which is not specifically limited here. The heating apparatus can rapidly increase the internal temperature of cryogenic physical therapy device to reduce the frostbite risk of a user at low-temperature during cryogenic physical therapy, and the heating apparatus can also be used for internal defrosting of cryogenic physical therapy device to improve refrigeration efficiency.

In one implementation, the target component can also include a variable air apparatus, which is used to control the air flow inside the cryogenic physical therapy device. Because the faster the air flow speed is, the lower the sensible temperature of the user is, so when the temperature is slightly higher, it is not necessary to spend a lot of energy through the refrigeration apparatus for refrigeration operation, but the sensible temperature of the user can be reduced by increasing the wind speed of the variable air apparatus. The variable air apparatus can also control the direction of air flow, allowing different parts of the body of a user to feel different temperatures. Because the sensitivity of the different parts of the body of the user to low temperature is different, it can better meet the cryogenic physical therapy needs of the user.

Furthermore, in step S503 above, the cryogenic physical therapy device can determine that when the user completes cryogenic physical therapy, it sends a fee deduction request to the server. The cryogenic physical therapy device can include a sensing apparatus, which may acquire internal environmental data of the cryogenic physical therapy device, including temperature data acquired by a temperature sensor, human body data detected by a human body sensor, monitoring videos acquired by a camera, and a cryogenic physical therapy duration acquired by a timer. Based on the above data, the cryogenic physical therapy device can determine the process of cryogenic physical therapy for the user, so that when the user completes cryogenic physical therapy, a fee deduction request can be sent to the server.

It can be seen that by using the solution provided by the embodiment of the present invention, the cryogenic physical therapy device can receive a use request sent by the server, control the target component to perform the operation corresponding to the use request, and send a fee deduction request to the server when determining that the cryogenic physical therapy has been completed by the user. After selecting a suitable cryogenic physical therapy device according to the actual situation, the cryogenic physical therapy device can control the target component to create an extremely low-temperature environment required for cryogenic physical therapy, and send a fee deduction request to the server when the cryogenic physical therapy has been completed by the user, thereby updating the account balance of the user. Therefore, the user does not need to go to a specific service merchant and can select a suitable cryogenic physical therapy device according to actual situations to perform cryogenic physical therapy quickly.

As one implementation of the present invention, as shown in Fig. 6, the step of controlling the target component to perform an operation corresponding to the use request can include:
S601, receiving authentication information of the user sent by the server.

In order to avoid use disputes caused by persons other than the user entering the cryogenic physical therapy device, or the risk of low-temperature frostbite caused by other persons mistakenly entering the cryogenic physical therapy device, the cryogenic physical therapy device can receive the authentication information of the user sent by the server. The authentication information is used to authenticate personnel entering the target cryogenic physical therapy device. For example, it can include biometric information such as facial feature information, fingerprint feature information, iris feature information, etc., it can also include password information provided by the server to the user in advance, information corresponding to the IC card, information corresponding to the RDFI tag, etc., which is not specifically limited here.

S602, acquiring target feature information of a target user and determining whether the target feature information matches the authentication information.

The cryogenic physical therapy device can acquire target feature information of a target user, the target user may be a person who wants to use the target cryogenic physical therapy device, including the user. In one implementation, the cryogenic physical therapy device may include a recognition apparatus that can collect target feature information of the target user. For example, when the authentication information is the fingerprint information of the user, the recognition apparatus can collect the fingerprint of the target user; when the authentication information is the password information provided by the server to the user in advance, the recognition apparatus can acquire the password input by the target user; and then the cryogenic physical therapy device can determine whether the target feature information of the target user matches the authentication information.

S603, if the target feature information matches the authentication information, controlling the target component to perform the operation corresponding to the use request.

If the target feature information of the target user matches the authentication information, it can be considered that the target user is the user. The target cryogenic physical therapy device can determine that the authentication has been passed, and can then control the target component to perform the operation corresponding to the use request. When the authentication fails, it can be considered that the target user is not the user, the target cryogenic physical therapy device does not control the target component to perform the operation corresponding to the use request.

It can be seen that in this embodiment, the cryogenic physical therapy device can receive the authentication information of the user sent by the server, acquire target feature information of the target user, and determine whether the target feature information matches the authentication information. If the target feature information matches the authentication information, the target component is controlled to perform the operation corresponding to the use request. Thus, the cryogenic physical therapy device can authenticate the target user, thereby avoid use disputes or even the risk of low-temperature frostbite caused by persons other than the user entering the cryogenic physical therapy device.

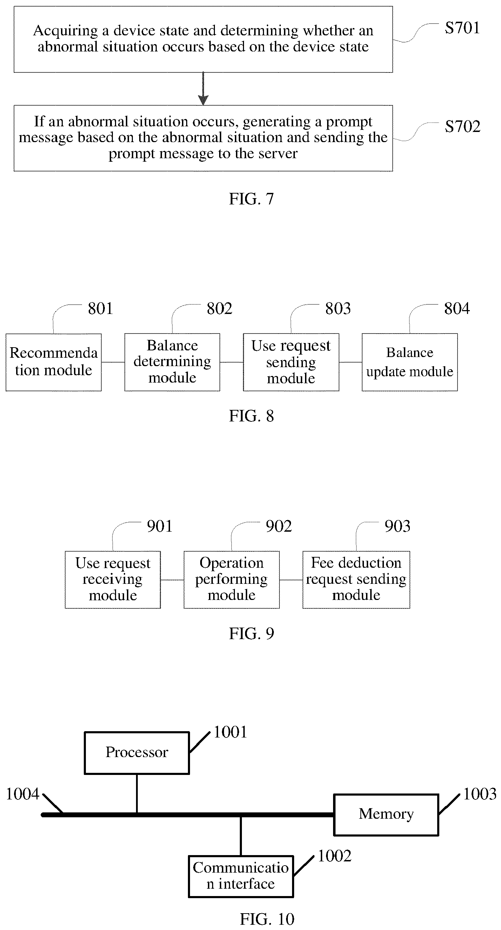

As one implementation of the present invention, as shown in Fig. 7, the above method may further include:
S701, acquiring a device state and determining whether an abnormal situation occurs based on the device state.

The cryogenic physical therapy device can acquire its own device state, the device state can include device information of the cryogenic physical therapy device, such as location information, device usage times, device fault information, etc., the device state can also include sensing data of the internal environment of the cryogenic physical therapy device acquired by the sensing apparatus, such as temperature data, humidity data, human body data, monitoring videos, cryogenic physical therapy duration, and other data.

Furthermore, based on the device state of the cryogenic physical therapy device, the cryogenic physical therapy device can determine whether an abnormal situation occurs, and the abnormal situation can include prolonged physiotherapy time for the user within the cryogenic physical therapy device, circuit failure of the cryogenic physical therapy device, etc. which is not specifically limited here.

S702, if an abnormal situation has occurred, generating a prompt message based on the abnormal situation and sending the prompt message to the server.

If an abnormal situation occurs, the cryogenic physical therapy device can generate a prompt message based on the abnormal situation. For example, when the abnormal situation is that a user has been in a cryogenic physical therapy device for too long, the prompt message can be "a user has been in a cryogenic physical therapy device A for too long". For example, when the abnormal situation is a circuit failure of a cryogenic physical therapy device, the prompt message can be "there is a circuit failure in the XX area of a cryogenic physical therapy device B".

Then the cryogenic physical therapy device can send the prompt message to the server, so that the server can generate a corresponding repair request based on the prompt message, and send the repair request to the device manufacturer of the cryogenic physical therapy device. Therefore, the device manufacturer of the cryogenic physical therapy device can quickly arrange staff for personnel rescue and device repair based on the repair request.

It can be seen that in this embodiment, the cryogenic physical therapy device can acquire the device state and determine whether an abnormal situation occurs based on the device state. If an abnormal situation occurs, a prompt message can be generated based on the abnormal situation and the prompt message is sent to the server. Therefore, the server can send a repair request to the device manufacturer of the cryogenic physical therapy device, so that the device manufacturer can quickly arrange staff for personnel rescue and device repair based on the repair request, in order to protect the physical health of the user and the property safety of the merchant.

Corresponding to the control method for the cryogenic physical therapy system applied to the server mentioned above, an embodiment of the present invention provides a control apparatus for a cryogenic physical therapy system.

As shown in Fig. 8, a control apparatus for a cryogenic physical therapy system is applied to a server in the cryogenic physical therapy system, the cryogenic physical therapy system further comprises cryogenic physical therapy devices, the apparatus includes:
a recommendation module 801, configured for acquiring target location information input by a user, and recommending a cryogenic physical therapy device to the user based on a relationship between the target location information and pre-recorded location information of each of the cryogenic physical therapy devices;
a balance determining module 802, configured for determining a target cryogenic physical therapy device selected by the user and determining whether an account balance of the user meets a preset fee deduction condition;
a use request sending module 803, configured for, if the account balance meets the preset fee deduction condition, sending a use request to the target cryogenic physical therapy device;
wherein the use request comprises a start time, a duration, and a physical therapy temperature;
a balance updating module 804, configured for receiving a fee deduction request sent by the target cryogenic physical therapy device and updating the account balance of the user based on the fee deduction request.

It can be seen that in the solution provided by the embodiment of the present invention, the server can acquire target location information input by a user, and recommend a cryogenic physical therapy device to the user based on a relationship between the target location information and pre-recorded location information of each of the cryogenic physical therapy devices; determine a target cryogenic physical therapy device selected by the user and determine whether an account balance of the user meets a preset fee deduction condition. If the account balance meets the preset fee deduction condition, a use request can be sent to the target cryogenic physical therapy device, wherein the use request includes a start time, a duration, and a physical therapy temperature. Furthermore, it can receive a fee deduction request sent by the target cryogenic physical therapy device and update the account balance of the user based on the fee deduction request. It can be seen that due to the pre-recorded location information of each of the cryogenic physical therapy devices, the server can recommend a cryogenic physical therapy device that is closer to the target location information after acquiring target location information input by a user, and the server can update the account balance of the user after completing the cryogenic physical therapy. Therefore, the user does not need to go to a specific service merchant and can select a suitable cryogenic physical therapy device according to actual situations to perform cryogenic physical therapy quickly.

As one implementation of the present invention, the recommendation module 801 may include:
an operating state acquisition unit, configured for acquiring an operating state of each of the cryogenic physical therapy devices;
wherein, the operating state includes a working state, a reserved state, and a standby state.
a first recommendation unit, configured for recommending a cryogenic physical therapy device whose operating state is the standby state to the user based on a relationship between the target location information and pre-recorded location information of each of the cryogenic physical therapy devices.

As one implementation of the present invention, the above apparatus may further include:
an operational information acquisition module, configured for acquiring operational information provided by a merchant of each of the cryogenic physical therapy devices;
wherein, the operational information includes business hours and prices for physical therapy of this cryogenic physical therapy device.

The above apparatus may further include:
a second recommendation unit, configured for, when recommending a cryogenic physical therapy device to the user, displaying the operational information corresponding to the recommended cryogenic physical therapy device.

As an implementation of the present invention, the above server can pre-record authentication information and an account balance of each user, and the above apparatus may further include:
an user information acquisition module, configured for acquiring feature information and/or recharge information provided by the user;
an user information update module, configured for updating the authentication information of the user based on the feature information, and updating the account balance of the user based on the recharge information.

The above apparatus may further include:
an authentication information sending unit, configured for sending, if the account balance meets the preset fee deduction condition, authentication information of the user to the target cryogenic physical therapy device after the step of determining a target cryogenic physical therapy device selected by the user.

As one implementation of the present invention, the above apparatus may further include:
a prompt message acquisition module, configured for receiving a prompt message sent by each of the cryogenic physical therapy devices;
wherein, the prompt message is generated when this cryogenic physical therapy device determines an abnormal situation occurs based on a device state;
a repair request sending module, configured for generating a repair request based on the prompt message and sending the repair request to a device manufacturer of the cryogenic physical therapy device.

As one implementation of the present invention, the above apparatus may further include:
an income distribution module, configured for, when the account balance of the user is updated based on the fee deduction request, performing income distribution between a merchant of the cryogenic physical therapy device and a device manufacturer of the cryogenic physical therapy device according to a preset ratio.

Corresponding to the control method for the cryogenic physical therapy system applied to the cryogenic physical therapy device mentioned above, an embodiment of the present invention provides a control apparatus for the cryogenic physical therapy system.

As shown in Fig. 9, a control apparatus for a cryogenic physical therapy system is applied to a cryogenic physical therapy device in the cryogenic physical therapy system, the cryogenic physical therapy system also comprises a server, the apparatus includes:
a use request receiving module 901, configured for receiving a use request sent by the server;
wherein the use request comprises a start time, a duration, and a physical therapy temperature;
an operation performing module 902, configured for controlling a target component to perform an operation corresponding to the use request;
a fee deduction request sending module 903, configured for, when determining that a user has completed cryogenic physical therapy, sending a fee deduction request to the server.

It can be seen that in the solution provided by the embodiment of the present invention, the cryogenic physical therapy device can receive a use request sent by the server, control the target component to perform an operation corresponding to the use request, and send a fee deduction request to the server when determining that the cryogenic physical therapy has been completed by the user. After selecting a suitable cryogenic physical therapy device according to the actual situation, the cryogenic physical therapy device can control the target component to create an extremely low-temperature environment required for cryogenic physical therapy, and send a fee deduction request to the server when the cryogenic physical therapy has been completed by the user, thereby updating the account balance of the user. Therefore, the user does not need to go to a specific service merchant and can select a suitable cryogenic physical therapy device according to actual situations to perform cryogenic physical therapy quickly.

As one implementation of the present invention, the operation performing module 902 may include:
an authentication information receiving unit, configured for receiving authentication information of the user sent by the server;
an information determining unit, configured for acquiring target feature information of a target user and determining whether the target feature information matches the authentication information;
an operation performing unit, configured for controlling, if the target feature information matches the authentication information, the target component to perform the operation corresponding to the use request.

As one implementation of the present invention, the above apparatus may also include:
an abnormal situation determining module, configured for acquiring a device state and determining whether an abnormal situation occurs based on the device state;
a prompt message sending module, configured for generating, if an abnormal situation occurs, a prompt message based on the abnormal situation and sending the prompt message to the server.

An embodiment of the present application provides an server, as shown in Fig. 10, which comprises a processor 1001, a communication interface 1002, a memory 1003 and a communication bus 1004, wherein the processor 1001, the communication interface 1002, and the memory 1003 communicate with each other via the communication bus 1004,
the memory 1003 is provided for storing a computer program;
the processor 1001 is used for performing the method of any one of embodiments above when executing the program stored on the memory 1003.

The communication bus described with respect to the server above can be a peripheral component interconnect (PCI) bus or an extended industry standard architecture (EISA) bus, and the like. The communication bus can include an address bus, a data bus, a control bus, or the like. For representation, only one thick line is shown in the figure, which does not mean there is only one communication bus or one type of communication bus.

The communication interfaces are used for communication between the server described above and the apparatuses.

The memory can include a random access memory (RAM), or can include a non-volatile memory (NVM), for example at least one disk memory. Optionally, the memory can also be at least one storage device located away from the processor described above.

The aforementioned processor can be a general-purpose processor, such as a central processing unit (CPU), a network processor (NP), or the like; it can also be a digital signal processor (DSP), an application specific integrated circuit (ASIC), a field-programmable gate array (FPGA) or other programmable logic device, a discrete gate or transistor logic device, a discrete hardware component.

Another embodiment provided by the present application provides a computer readable storage medium. The computer readable storage medium stores the computer program thereon, and a processor implements the method of any one of embodiments above when executing the computer program.

In still another embodiment according to the present invention, there is also provided a computer program product containing instructions that, when running on a computer, cause the computer to perform the method of any one of embodiments above.

In the aforementioned embodiments, it may be implemented in whole or in part by software, hardware, firmware, or any combination thereof. When implemented by software, it may be implemented in whole or in part in the form of a computer program product. The computer program product includes one or more computer instructions. The processes or functions described in accordance with the embodiments of the present invention is produced in whole or in part, when the computer program instructions are loaded and executed on a computer. The computer may be a general-purpose computer, a dedicated computer, a computer network, or other programmable device. The computer instructions may be stored in a computer-readable storage medium or may be transmitted from one computer-readable storage medium to another computer-readable storage medium, for example, the computer instructions may be transmitted from a web site, a computer, a server, or a data center to another web site, another computer, another server, or another data center via a cable (such as a coaxial cable, an optical fiber, a digital subscriber line (DSL)) or wireless (such as infrared, wireless, microwave, etc.). The computer-readable storage medium may be any available medium that may be accessed by a computer or a data storage device such as a server or a data center containing one or more available medium integrations. The available media may be magnetic media (such as floppy disks, hard disks, magnetic tapes), optical media (such as DVDs), or semiconductor media (such as solid state disk (SSD)), etc.

It should be noted that, the relationship terms herein such as "first", "second" and the like are only used to distinguish one entity or operation from another entity or operation, but do not necessarily require or imply that there is actual relationship or order between these entities or operations. Moreover, the terms "include", "comprise" or any other variants thereof are intended to cover non-exclusive inclusions, so that processes, methods, articles or devices comprising a series of elements comprise not only those elements listed but also those not specifically listed or the elements intrinsic to these processes, methods, articles, or devices. Without further limitations, elements defined by the sentences "comprise(s) a." or "include(s) a." do not exclude that there are other identical elements in the processes, methods, articles, or devices which include these elements.

All the embodiments are described in corresponding ways, same or similar parts in each of the embodiments can be referred to one another, and the parts emphasized are differences to other embodiments. In particular, for embodiments of the apparatus, server, computer readable storage medium, and computer program product, since they are substantially similar to the embodiments of the method, their description is relatively simple, and for the related aspects, one only needs to refer to portions of the description of the embodiments of the method.

The embodiments described above are just preferred embodiments of the disclosure, and not intended to limit the scope of the disclosure. Any modifications, equivalent, improvement or the like within the spirit and principle of the disclosure should be included in the scope of the disclosure.

## Claims

1. A control method for a cryogenic physical therapy system, wherein the method is applied to a server in the cryogenic physical therapy system, the cryogenic physical therapy system further comprises cryogenic physical therapy devices, the method comprises:
acquiring target location information input by a user, and recommending a cryogenic physical therapy device to the user based on a relationship between the target location information and pre-recorded location information of each of the cryogenic physical therapy devices;
determining a target cryogenic physical therapy device selected by the user and determining whether an account balance of the user meets a preset fee deduction condition;
if the account balance meets the preset fee deduction condition, sending a use request to the target cryogenic physical therapy device to enable the target cryogenic physical therapy device to control a target component to perform an operation corresponding to the use request, wherein the use request comprises a start time, a duration, and a physical therapy temperature;
receiving a fee deduction request sent by the target cryogenic physical therapy device and updating the account balance of the user based on the fee deduction request.

2. The method of claim 1, wherein the step of recommending a cryogenic physical therapy device to the user based on a relationship between the target location information and pre-recorded location information of each of the cryogenic physical therapy devices comprise:
acquiring an operating state of each of the cryogenic physical therapy devices, wherein the operating state comprises a working state, a reserved state, and a standby state;
recommending a cryogenic physical therapy device whose operating state is the standby state to the user based on the relationship between the target location information and pre-recorded location information of each of the cryogenic physical therapy devices.

3. The method of claim 1, further comprising:
acquiring operational information provided by a merchant of each of the cryogenic physical therapy devices, wherein the operational information comprises business hours and prices for physical therapy of this cryogenic physical therapy device;
the method further comprises:
when recommending a cryogenic physical therapy device to the user, displaying the operational information corresponding to the recommended cryogenic physical therapy device.

4. The method of claim 1, wherein the server pre-records authentication information and an account balance of each user, and the method further comprises:
acquiring feature information and/or recharge information provided by the user;
updating the authentication information of the user based on the feature information, and updating the account balance of the user based on the recharge information;
after the step of determining a target cryogenic physical therapy device selected by the user, the method further comprises:
if the account balance meets the preset fee deduction condition, sending the authentication information of the user to the target cryogenic physical therapy device to enable the target cryogenic physical therapy device to authenticate a target user.

5. The method of claim 1, further comprising:
receiving a prompt message sent by a cryogenic physical therapy device, wherein the prompt message is generated when the cryogenic physical therapy device determines that an abnormal situation occurs based on a device state; generating a repair request based on the prompt message and sending the repair request to a device manufacturer of the cryogenic physical therapy device;
or wherein the method further comprises: when the account balance of the user is updated based on the fee deduction request, performing income distribution between a merchant of the target cryogenic physical therapy device and a device manufacturer of the target cryogenic physical therapy device according to a preset ratio.

6. A control method for a cryogenic physical therapy system, wherein the method is applied to a cryogenic physical therapy device in the cryogenic physical therapy system, the cryogenic physical therapy system further comprises a server, the method comprises:
receiving a use request sent by the server, wherein the use request comprises a start time, a duration, and a physical therapy temperature;
controlling a target component to perform an operation corresponding to the use request;
when determining that a user has completed cryogenic physical therapy, sending a fee deduction request to the server.

7. The method of claim 6, wherein the step of controlling a target component to perform an operation corresponding to the use request comprise: receiving authentication information of the user sent by the server; acquiring target feature information of a target user and determining whether the target feature information matches the authentication information; if the target feature information matches the authentication information, controlling the target component to perform the operation corresponding to the use request;
or wherein the method further comprises: acquiring a device state and determining whether an abnormal situation occurs based on the device state; if an abnormal situation occurs, generating a prompt message based on the abnormal situation and sending the prompt message to the server.

8. A control apparatus for a cryogenic physical therapy system, wherein the apparatus is applied to a server in the cryogenic physical therapy system, the cryogenic physical therapy system further comprises cryogenic physical therapy devices, the apparatus comprises:
a recommendation module, configured for acquiring target location information input by a user, and recommending a cryogenic physical therapy device to the user based on a relationship between the target location information and pre-recorded location information of each of the cryogenic physical therapy devices;
a balance determining module, configured for determining a target cryogenic physical therapy device selected by the user and determining whether an account balance of the user meets a preset fee deduction condition;
a use request sending module, configured for sending, if the account balance meets the preset fee deduction condition, a use request to the target cryogenic physical therapy device to enable the target cryogenic physical therapy device to control a target component to perform an operation corresponding to the use request, wherein the use request comprises a start time, a duration, and a physical therapy temperature;
a balance updating module, configured for receiving a fee deduction request sent by the target cryogenic physical therapy device and updating the account balance of the user based on the fee deduction request.

9. The apparatus of claim 8, wherein the recommendation module comprises: an operating state acquisition unit, configured for acquiring an operating state of each of the cryogenic physical therapy devices, wherein the operating state comprises a working state, a reserved state, and a standby state; a first recommendation unit, configured for recommending a cryogenic physical therapy device whose operating state is the standby state to the user based on the relationship between the target location information and pre-recorded location information of each of the cryogenic physical therapy devices;
or wherein the apparatus further comprises: an operational information acquisition module, configured for acquiring operational information provided by a merchant of each of the cryogenic physical therapy devices, wherein the operational information comprises business hours and prices for physical therapy of this cryogenic physical therapy device; a second recommendation unit, configured for, when recommending a cryogenic physical therapy device to the user, displaying the operational information corresponding to the recommended cryogenic physical therapy device.

10. The apparatus of claim 8, wherein the server pre-records authentication information and an account balance of each user, and the apparatus further comprises:
an user information acquisition module, configured for acquiring feature information and/or recharge information provided by the user;
an user information update module, configured for updating the authentication information of the user based on the feature information, and updating the account balance of the user based on the recharge information;
the apparatus further comprises:
an authentication information sending unit, configured for sending, if the account balance meets the preset fee deduction condition, authentication information of the user to the target cryogenic physical therapy device to enable the target cryogenic physical therapy device to authenticate a target user after determining the target cryogenic physical therapy device selected by the user.

11. The apparatus of claim 8, further comprising: a prompt message acquisition module, configured for receiving a prompt message sent by a cryogenic physical therapy device, wherein the prompt message is generated when the cryogenic physical therapy device determines that an abnormal situation occurs based on a device state; a repair request sending module, configured for generating a repair request based on the prompt message and sending the repair request to a device manufacturer of the cryogenic physical therapy device;
or wherein the apparatus further comprises: an income distribution module, configured for, when the account balance of the user is updated based on the fee deduction request, performing income distribution between a merchant of the target cryogenic physical therapy device and a device manufacturer of the target cryogenic physical therapy device according to a preset ratio.

12. A control apparatus for a cryogenic physical therapy system, wherein the apparatus is applied to a cryogenic physical therapy device in the cryogenic physical therapy system, the cryogenic physical therapy system further comprises a server, the apparatus comprises:
a use request receiving module, configured for receiving a use request sent by the server, wherein the use request includes a start time, a duration, and a physical therapy temperature;
an operation performing module, configured for controlling a target component to perform an operation corresponding to the use request;
a fee deduction request sending module, configured for, when determining that a user has completed cryogenic physical therapy, sending a fee deduction request to the server.

13. The apparatus of claim 12, wherein the operation performing module comprises: an authentication information receiving unit, configured for receiving authentication information of the user sent by the server; an information determining unit, configured for acquiring target feature information of a target user and determining whether the target feature information matches the authentication information; an operation performing unit, configured for controlling, if the target feature information matches the authentication information, the target component to perform the operation corresponding to the use request;
or wherein the apparatus further comprises: an abnormal situation determining module, configured for acquiring a device state and determining whether an abnormal situation occurs based on the device state; a prompt message sending module, configured for generating, if an abnormal situation occurs, a prompt message based on the abnormal situation and sending the prompt message to the server.

14. A server comprising a processor, a communication interface, a memory and a communication bus; wherein the processor, the communication interface and the memory communicate with each other via the communication bus;
the memory is provided for storing a computer program;
the processor is used for implementing the method of any one of claims 1-5 when executing the program stored on the memory.

15. A computer readable storage medium, wherein the computer readable storage medium stores a computer program thereon, and the method of any one of claims 1-5 or 6-7 is implemented when the computer program is executed by a processor.
